# EUROPEAN PATENT APPLICATION

(11) **EP 4 610 343 A1**
(43) Date of publication of application: **03.09.2025**
(21) Application number: 24197900.4
(22) Date of filing: 02.09.2024
(51) Int. Cl.: C12M 1/24, C12M 1/04, C12M 1/00

(54) **AUTOMATIC CULTURE DEVICE AND CELL CULTURE METHOD USING SAME**

(30) Priority: 01.03.2024 JP 2024031221
(71) Applicant: HITACHI HIGH-TECH CORPORATION, Tokyo 105-6409 (JP)
(72) Inventor: KIYAMA, Masaharu, Tokyo, 100-8280 (JP); HIRAI, Kakuro, Toyko, 100-8280 (JP); KOBAYASHI, Toyoshige, Tokyo, 100-8280 (JP); ABE, Makoto, Kanagawa, 220-6122 (JP)
(74) Representative: MERH-IP Matias Erny Reichl Hoffmann Patentanwälte PartG mbB

(57) **Abstract**

The invention provides an automatic culture device that implements maintenance of a CO₂ gas concentration of a culture vessel necessary for culture with a simple configuration, and a cell culture method using the automatic culture device.

An automatic culture device includes: a ventilation adapter configured to supply, in a state in which a culture vessel configured to culture cells is placed thereon, a gas containing CO₂ to the culture vessel; a gas supply unit configured to supply the gas containing CO₂ to the ventilation adapter; and a gas holding space forming portion configured to airtightly hold the gas containing CO₂ supplied from the gas supply unit when the culture vessel is placed at a predetermined position of the ventilation adapter, an upper portion of the gas holding space forming portion directly or indirectly supporting at least a part of a gas exchange membrane provided on a lower side of the culture vessel, and a lower end portion of the gas holding space forming portion coming into contact with at least a part of an upper surface of the ventilation adapter.

## Description

### Technical Field

The present invention relates to an automatic culture device for culturing cells or tissues, and a cell culture method using the same.

### Background Art

A medical treatment using regenerated human cells and cells with modified functions through gene transfer can overcome diseases that have been difficult to cure so far. Therefore, the medical treatment is expected to be widely used.

The most common treatment target is a cancer, followed by various organs. This is considered to be because autologous transplants using the cells of patients have a low possibility of rejection and are in high demand from the perspective of improving patient quality of life (QOL).

The most common cell type is immune cells, accounting for more than 40% of the total. The main target disease is cancer, and specific cell types include T cells, NK cells, NKT cells, and the like. In particular, the practical application of chimeric antigen receptor-T (CAR-T) cell therapy, in which T cells, which are immune cells, are extracted from a patient, processed by gene transfer into a form capable of attacking cancer cells, and then returned to the patient by injection, is currently at the forefront.

In a step of producing cells for transplantation, a biological sample taken from a patient or another person is separated and purified and subjected to processing such as amplification and genetic introduction.

This step is carried out in a cell processing center (CPC) according to a standard operating procedure (SOP) satisfying a good manufacturing practice (GMP) which is a standard for manufacturing management and quality management of pharmaceutical products and the like. Therefore, the operation of the CPC requires significant costs and personnel with specialized culture techniques.

In addition, since the manufacturing step is mainly performed manually, there is a limit to an increase in the manufacturing amount. The low productivity and the high manufacturing costs are impeding the widespread adoption of regenerative medicine, and there is a demand for automation of a culture operation that particularly requires labor and costs in a manufacturing step. It is possible to save labor, reduce costs, and achieve mass production due to automation of culture work.

Examples of an automatic culture device include a device in which a closed flow path having a closed space is automatically handled as shown in PTL 1. In the closed flow path, a closed culture vessel is always connected to a flow path tube or the like, and cells are cultured inside the closed culture vessel.

The closed flow path enables the movement of a liquid and a gas held therein by an operation of a valve, a pump, and the like proposed outside the closed flow path. Accordingly, the automatic culture device automatically performs cell seeding, culture medium change, microscopic observation, and the like while maintaining the closing property of the culture space.

As an example of the culture vessel, as shown in PTL 2, there is a technique in which a closed vessel having a ventilation membrane in a part of the culture vessel is used, and the periphery of the ventilation surface is maintained at a desired gas concentration to perform cell culture.

### Citation List

### Patent Literature

PTL 1: JP2007-312668A
PTL 2: US4,839,292A

### Summary of Invention

### Technical Problem

In the technique described in PTL 1, culture can be performed in a closed culture vessel. However, in order to perform culture, the culture vessel is held in a CO₂ incubator whose inside is maintained at a constant CO₂ gas concentration. Gas exchange is performed by dissolving a gas in a space present above the culture medium in the culture vessel into the culture medium or releasing a gas in the culture medium into the space.

In addition, the water held inside the CO₂ incubator for humidification is brought into contact with the atmosphere, and thus, there is a risk of microorganism generation here, and there are operational hurdles to overcome when using cells for transplantation in manufacturing facilities, where high levels of cleanliness are required.

In PTL 2, a ventilation chamber is provided to maintain the periphery of a ventilation surface of a culture vessel at a desired gas concentration without using a CO₂ incubator. The inside of the ventilation chamber has a positive pressure due to a pressure loss of the pipeline or the like, and therefore, if a ventilation chamber having high airtightness cannot be provided, the humidified gas leaks out from the periphery of the culture vessel and leaks to the inside of the incubator to increase the humidity of the inside of the waste, which may cause mold or the like. However, this point is not mentioned in PTL 2.

An object of the invention is to provide an automatic culture device that implements maintenance of a CO₂ gas concentration of a culture vessel necessary for culture with a simple configuration, and a cell culture method using the automatic culture device.

### Solution to Problem

A configuration of the invention for achieving the above object is as follows.

An automatic culture device includes: a ventilation adapter configured to supply, in a state in which a culture vessel configured to culture cells is placed thereon, a gas containing CO₂ to the culture vessel; a gas supply unit configured to supply the gas containing CO₂ to the ventilation adapter; and a gas holding space forming portion configured to airtightly hold the gas containing CO₂ supplied from the gas supply unit when the culture vessel is placed at a predetermined position of the ventilation adapter, an upper portion of the gas holding space forming portion directly or indirectly supporting at least a part of a gas exchange membrane provided on a lower side of the culture vessel, and a lower end portion of the gas holding space forming portion coming into contact with at least a part of an upper surface of the ventilation adapter.

### Advantageous Effects of Invention

The invention can provide an automatic culture device that implements maintenance of a CO₂ gas concentration of a culture vessel necessary for culture with a simple configuration, and a cell culture method using the automatic culture device.

### Brief Description of Drawings

[FIG. 1] FIG. 1 is a view showing a configuration of an automatic culture device.
[FIG. 2] FIG. 2 is a view showing an example of a ventilation adapter.
[FIG. 3] FIG. 3 is a view showing a result of gas exchange in a culture vessel by the ventilation adapter.
[FIG. 4] FIG. 4 is a view showing an example of a pressing jig provided in the ventilation adapter.
[FIG. 5] FIG. 5 is a view showing an example of a pressing jig provided in the ventilation adapter.
[FIG. 6] FIG. 6 is a view showing an example of a pressing jig provided in the ventilation adapter.
[FIG. 7] FIG. 7 is a view showing an example of a gas holding space forming portion provided in the ventilation adapter.
[FIG. 8] FIG. 8 is a view showing an example of a gas holding space forming portion provided in the ventilation adapter.
[FIG. 9] FIG. 9 is a diagram showing a flow during operation of the automatic culture device.
[FIG. 10A] FIG. 10A is a view showing an example of a flow path circuit including a closed culture vessel.
[FIG. 10B] FIG. 10B is a view showing an example of a procedure of cell seeding.
[FIG. 10C] FIG. 10C is a view showing an example of a procedure of gas exchange.
[FIG. 10D] FIG. 10D is a view showing an example of a procedure of culture medium addition.
[FIG. 10E] FIG. 10E is a view showing an example of a procedure of culture medium change.
[FIG. 10F] FIG. 10F is a diagram showing an example of a procedure of supernatant sampling.
[FIG. 10G] FIG. 10G is a diagram showing an example of a procedure of cell collection.

### Description of Embodiments

Hereinafter, embodiments of the invention will be described with reference to the drawings. The following description shows a specific example of the content of the invention, and the invention is not limited to the description, and various modifications and modifications can be made by those skilled in the art within the scope of the technical idea disclosed in the description.

In addition, in all the drawings for illustrating the invention, components having the same functions are denoted by the same reference signs, and repeated description thereof may be omitted.

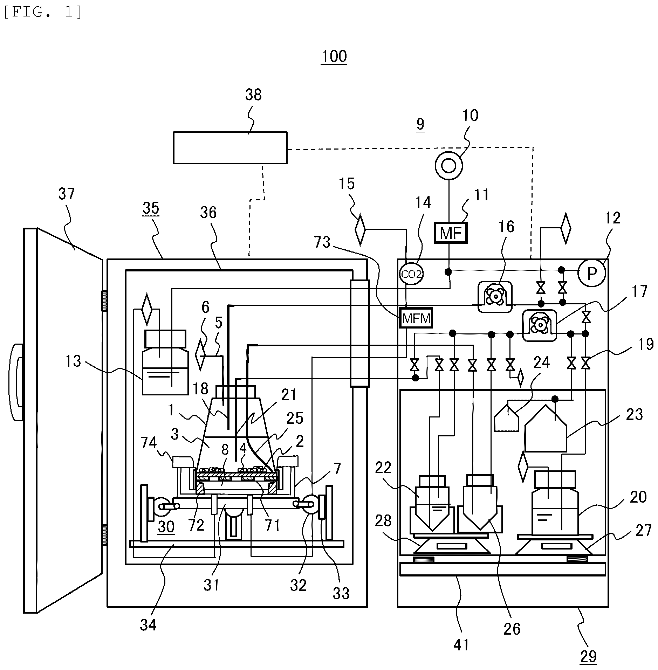

Components of an automatic culture device of the present embodiment which performs culture using a closed culture vessel will be described with reference to FIG. 1.

The automatic culture device 100 includes a culture vessel 1, a ventilation adapter 7, a gas supply unit 9, a pump for feeding a liquid or a gas, flow paths for connecting the components, valves for opening and closing the flow paths, a control unit 38 for controlling the gas supply unit, the pump, the valves, and the like, a swing mechanism 30 for swinging the culture vessel 1, and an incubator 35 as a temperature maintenance mechanism for accommodating the culture vessel, the ventilation adapter, the swing mechanism, and the like and performing temperature control. The culture vessel 1 is a ventilation surface-attached culture vessel having a ventilation surface (also referred to as a gas exchange membrane) 4, a bottom surface of which is provided with a gas permeable membrane, and a perforated bottom surface 71 is in contact with a lower surface of the culture vessel 1. The perforated bottom surface 71 has a function of supporting the ventilation surface 4, which is a thin film, from below in a horizontal plane, a ventilation function for ventilation by a plurality of holes, and a function of preventing downward leakage of a liquid by being in airtight contact with an outer periphery of the ventilation surface 4.

An upper end portion of the gas holding space forming portion 72 is in contact with a lower surface of the perforated bottom surface 71 so as to indirectly support the ventilation surface, and a lower end portion thereof is in contact with an upper surface (inner surface) of the ventilation adapter 7, thereby forming a ventilation space 8 that can be airtightly maintained at a desired gas concentration. The term "airtight" as used herein means that a gas supplied from the gas supply unit does not leak out of the ventilation space 8, and the term does not mean that the gas is confined in the ventilation space 8 and the confined gas stagnates (does not flow).

In the culture vessel 1, cells 2 are cultured while being held together with a culture medium 3. A pressure regulating pipe 5 and a vent filter 6 are connected to the culture vessel 1, and thus a gas inside the culture vessel can flow in and out, and the entry of bacteria and viruses from the outside is prevented.

In addition, the culture vessel 1 is attached to the ventilation adapter 7, and gas supply and humidification necessary for culture can be performed to the culture vessel 1. The gas supply unit 9 includes a gas cylinder 10 in which a predetermined gas concentration is maintained, a gas flow rate control unit (mass flow controller: MF) 11, a pressure sensor 12, and a humidification bottle 13 serving as a humidification unit, and is connected to an upstream side of the ventilation adapter 7. For the gas supply and humidification, a gas is fed by controlling an air supply amount to a predetermined air supply amount with the gas flow rate control unit, and is humidified by passing through water inside the humidification bottle and then is supplied to the ventilation adapter 7. A gas flow meter (mass flow meter: MFM) 73, a CO₂ sensor 14, and a CO₂ gas vent filter 15 are connected downstream of the ventilation adapter 7, and the gas is discharged to the atmosphere outside the device. The gas flow meter 73 and the CO₂ sensor 14 can monitor, by a flow rate of the gas and a measured value of CO₂, whether the gas exchange in the ventilation adapter is appropriately performed, and can be used for prediction of a culture state.

The feeding of a liquid or the feeding of a gas is performed by pumps 16 and 17, and a preferable pump is a tube pump that generates pressure by squeezing a rubber tube with a rotating roller. The pump 16 is configured to feed a culture medium to the culture vessel 1 and is connected to a liquid feeding pipe 18 of the culture vessel 1 by a tube. On the other hand, the pump 16 is connected to a supply pipe of a culture medium bottle 20 via a solenoid valve 19.

A solenoid valve is preferable as a valve for opening and closing a rubber tube constituting the flow path, and when electricity is applied, the solenoid valve is operated to open the rubber tube in the closed state which is clamped by the valve operated by a spring force.

The pump 17 is configured to feed a cell suspension to the culture vessel 1 to perform cell seeding, or discharge the culture medium 3 from the culture vessel 1 to collect grown cells.

The pump 17 is connected to an aspirating tube (also referred to as a "liquid discharge pipe") 21 of the culture vessel 1 by a tube. On the other hand, the pump 17 is connected to a cell seeding bottle 22 via a solenoid valve 19 and is connected to a supernatant collection bag 23 and a supernatant analysis bag 24 via another solenoid valve 19.

The pump 17 is connected to a collection pipe 25 for cells in the culture vessel 1 by a tube, and on the other hand, is connected to a cell collection bottle 26 via a solenoid valve 19.

The weight of the culture medium bottle 20 is measured by a weight sensor 27, and the weight of the cell seeding bottle 22 and the weight of the cell collection bottle 26 are measured by a weight sensor 28.

As shown in FIG. 1, the culture medium bottle, the cell seeding bottle, the cell collection bottle, the supernatant collection bag, the supernatant analysis bag, the flow path connecting the components, the pump, the solenoid valve, and the weight sensor are provided in a fluid control unit 29 which is a device body outside the incubator 35.

The swing mechanism 30 includes a swing stage 31 for holding the ventilation adapter 7, link mechanisms 32 for supporting the swing stage from three directions, swing shafts 33 respectively connected to the link mechanisms 32, and a swing stage 34 fixed to an inside of the incubator.

In a swing operation of the culture vessel, when control is performed such that the right swing shaft is lowered downward, the left swing shaft is raised upward simultaneously, and the central swing shaft is not moved, the swing stage moves with a tilt, and the culture vessel can be tilted. Next, by reversing motion of the left and right shafts, the culture vessel can be tilted in an opposite direction.

When the swing shafts are continuously operated and the swing shaft is also moved in a depth direction of the paper, the culture vessel can be tilted back and forth, and therefore, the cells 2 and the culture medium 3 inside the culture vessel 1 can be stirred.

The incubator 35 is an example of a temperature maintaining mechanism, and is a so-called dry incubator including a constant temperature unit 36 and an opening/closing door 37. The incubator 35 can house the culture vessel 1, the swing mechanism 30, and the humidification bottle 13, and can maintain the inside of the incubator at a temperature suitable for cell culture. By adopting a small-sized dry incubator in place of a large-sized CO₂ incubator and having a configuration capable of performing gas supply necessary for culture, the device can be downsized, and a plurality of devices can be simultaneously operated in a small space.

The control unit 38 can control the operations of the gas supply unit 9, the pumps 16 and 17, and the solenoid valve 19, and the operation of the swing mechanism 30. By automatically controlling the mechanical elements at a predetermined timing, the cell suspension can be fed from the cell seeding bottle 22 to the culture vessel 1 during cell seeding, the gas humidified by the humidification bottle 13 can be supplied to the ventilation adapter 7 during gas exchange, the culture medium can be fed from the culture medium bottle 20 to the culture vessel 1 during culture medium addition, a culture medium can be supplied to the culture vessel 1 after the culture medium 3 in the culture vessel 1 is discharged to the supernatant collection bag 23 during culture medium change, and a part of the culture medium in the culture vessel can be fed to the supernatant analysis bag 24 during supernatant sampling.

During cell collection, after the culture medium in the culture vessel 1 is discharged to the supernatant collection bag 23, the cell suspension can be stirred by operating the swing mechanism 30 and then fed to the cell collection bottle 26.

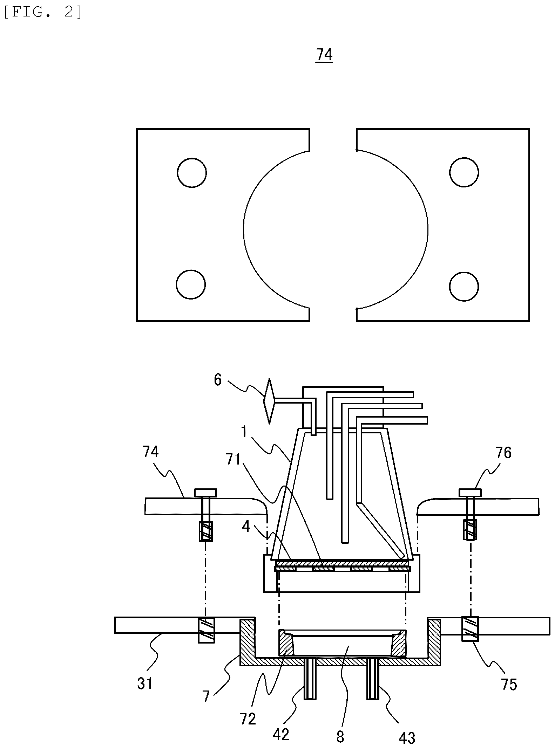

FIG. 2 is a schematic assembly view of the ventilation adapter 7 and the culture vessel 1. The gas holding space forming portion 72 is fixed at a predetermined position on the upper surface of the ventilation adapter 7. In this embodiment, the gas holding space forming portion 72 is entirely formed of rubber having flexibility. The gas holding space forming portion 72 is fixed using a method by which the gas holding space forming portion 72 can be easily removed when the rubber forming the gas holding space forming portion 72 is consumed and replaced. Next, the culture vessel 1 is inserted from above so as to conform to an outer shape of the gas holding space forming portion 72.

The ventilation surface 4 and the perforated bottom surface 71 of the culture vessel 1 are surrounded by a skirt portion (which is generally made of hard rubber, a synthetic resin, or the like, but may be made of metal such as aluminum, stainless steel, or the like) that covers a lower portion of the culture vessel 1. The expression "inserted so as to conform to the outer shape" means that the culture vessel 1 is inserted such that the gas holding space forming portion 72 is in airtight contact with an inner peripheral surface of the skirt portion.

At this time, a height of the gas holding space forming portion 72 is a natural length. Next, one end of the pressing jig 74 is temporarily placed in contact with an outer surface shape of the culture vessel 1. A fastener receiver 75 is provided on the stage 31, and the fastener receiver 75 engages with a fastener 76 at a suitable position of the pressing jig 74. The pressing jig 74 includes two components that are placed symmetrically with respect to the vessel. When the fastener is rotated and engaged, the pressing jig 74 is pulled downward and presses the culture vessel 1 downward. At this time, the gas holding space forming portion 72 is pressed down by the perforated bottom surface 71 of the culture vessel 1 in the same manner, is sealed closely by elastic deformation of the rubber, and comes into close contact with the ventilation adapter 7. Then, the ventilation surface 4 receives a force from the gas holding space forming portion 72 via the perforated bottom surface 71, that is, indirectly, and the ventilation surface 4 is deformed to reduce a gap between the ventilation surface 4 and other members, thereby forming the airtight ventilation space 8. The CO₂ gas is supplied from a gas feeding pipe 42 provided on a bottom surface of the ventilation adapter 7 and is discharged from a port 43 after the CO₂ gas comes into contact with the gas permeable membrane 4 through the ventilation space 8.

In the method for placing the pressing jig 74, after the culture vessel 1 is placed on the ventilation adapter 7, the pressing jig 74 is placed on each of the left and right sides of the culture vessel 1. The feature of the pressing jig is that the pressing jig can be placed without interfering with four tubes connected to the culture vessel 1. In addition, when two plates are produced in the same shape, the plate can be placed on either the left side or the right side.

According to the above configuration, since the ventilation surface 4 can be formed with high airtightness together with the ventilation adapter 7 and the gas holding space forming portion 72 via the perforated bottom surface 71, the humidified CO₂ gas is held in the ventilation space 8 and does not leak into the incubator, so that the incubator can be maintained at low humidity.

An upper portion of the gas holding space forming portion 72 may be in direct contact with the ventilation surface 4, that is, may directly support the ventilation surface 4. In this case, the ventilation surface 4 directly receives a force from the gas holding space forming portion 72, and the ventilation surface 4 is deformed to reduce a gap between the ventilation surface 4 and other members, thereby forming the airtight ventilation space 8.

Further, the humidified CO₂ gas is continuously supplied to an internal space of the ventilation adapter 7, and the internal space (ventilation space 8) of the ventilation adapter 7 is maintained at a pressure slightly higher than the atmospheric pressure due to the pressure loss caused by passing through the CO₂ vent filter 15 shown in FIG. 1. That is, the internal space of the ventilation adapter 7 has a positive pressure compared with the atmospheric pressure. Accordingly, the risk that bacteria in the atmosphere enter the internal space of the ventilation adapter 7 is reduced.

A volume of the internal space of the ventilation adapter 7 is preferably as small as possible as long as the humidified CO₂ gas can be uniformly supplied to the gas permeable membrane of the culture vessel. This is because when the volume of the internal space is large, the time for filling the internal space with the humidified CO₂ gas becomes long.

On the other hand, when the internal space is too small, the humidified CO₂ gas may not be supplied uniformly. Therefore, it is preferable that the internal space is a space that is as thin as possible in an up-down direction and has a shape having an area equal to or slightly larger than an area of the gas permeable membrane of the culture vessel in a horizontal direction. The space shape is circular when the vessel is cylindrical, and is square when the vessel is rectangular.

In the gas holding space forming portion 72, only the upper end portion and the lower end portion of the gas holding space forming portion 72 for maintaining airtightness may be made of a material having flexibility such as rubber, and an intermediate portion may be made of metal, hard plastic, or the like.

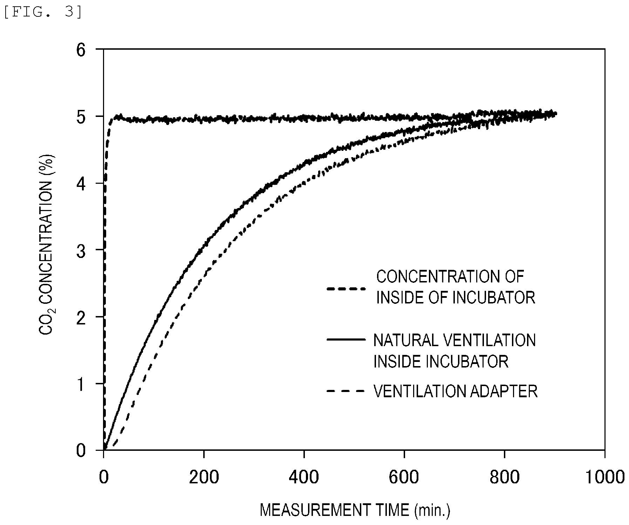

FIG. 3 shows the results of comparing the temporal changes in concentrations of the CO₂ gas as a gas phase in the culture vessel in Embodiment 1 using two measurement methods, a ventilation method using the ventilation adapter 7 and a ventilation method using a 5% CO₂ incubator (the technology described in PTL 1).

For the measurement based on the ventilation method using the ventilation adapter 7, a CO₂ sensor (VAISSLA GM70 handy type CO₂ meter) (not shown) was placed inside the closed culture vessel 1, the vent filter 6 was temporarily closed, and a vessel in which a gas passes only through the ventilation surface (gas permeable membrane) 4 was produced. The volume of the ventilation space 8 was 300 cc, and a mixed gas of 5% CO₂-air was continuously fed at a flow rate of 50 cc/min from the gas feeding pipe 42.

In the measurement based on the ventilation method using the CO₂ incubator, a CO₂ sensor was placed inside the incubator always maintained with 5% CO₂-air, a separate CO₂ sensor was placed inside the closed culture vessel 1 simultaneously, and a vessel in which a gas passes only the ventilation surface (gas permeable membrane) 4 was produced. The two vessels were maintained at 37°C, and measurements were performed for 15 hours.

As a result, the 5% CO₂ concentration was reached in 800 minutes in both the ventilation method using the CO₂ incubator and the ventilation method using the ventilation adapter.

In the ventilation using the ventilation adapter, the same ventilation as the ventilation using the CO₂ incubator can be performed by adjusting the gas feeding amount in consideration of the fact that it takes time to increase the gas concentration of the ventilation space 8.

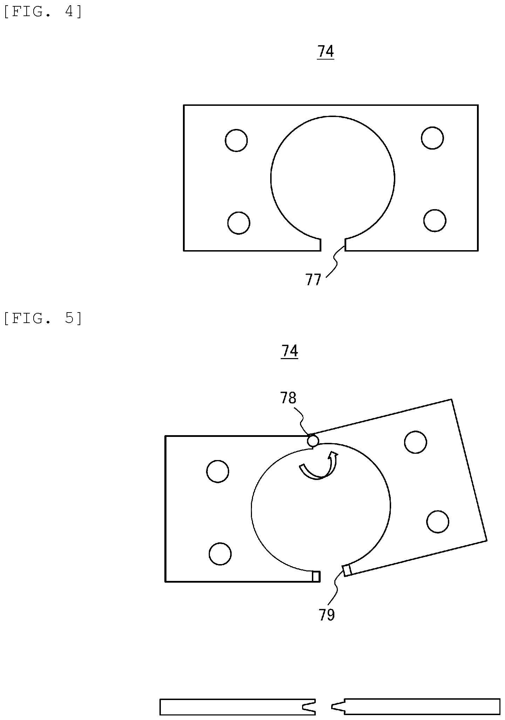

FIG. 4 shows another embodiment of the pressing jig 74. A feature of the pressing jig 74 is that an opening 77 is provided in a central portion through which the culture vessel 1 penetrates. In the placement method, after the culture vessel 1 is placed on the ventilation adapter 7, four tubes pass through the opening 77 above the culture vessel 1, and then the pressing jig 74 is placed on the swing stage. The feature of the pressing jig 74 is that the pressing jig 74 can be produced by a single plate, is advantageous in terms of costs, and can press the vessel horizontally with a constant force. Further, the pressing jig 74 can be placed even if the depth direction is reversed.

FIG. 5 shows another embodiment of the pressing jig 74. A feature of the pressing jig 74 is that the pressing jig 74 has an opening 79 that can be opened and closed around a rotation shaft 78. The shapes of left and right end portions of the opening 79 are a recessed shape and a protruding shape fitting into each other, and when the pressing jig 74 is closed during the placement, the left and right end portions are integrated to increase the horizontal strength.

In the placement method, after the culture vessel 1 is placed in the ventilation adapter 7, the pressing jig 74 is opened on a girth side of the culture vessel 1 and is closed after the vessel passes therethrough. Next, the pressing jig 74 is placed on the swing stage. The feature of the pressing jig 74 is that the pressing jig 74 can be produced by a set of components, and when the pressing jig 74 is closed, the pressing jig 74 can be in contact with the vessel at equal intervals on a circumference of the vessel and press the vessel horizontally with a constant force. The pressing jig 74 can be placed even if the depth direction is reversed.

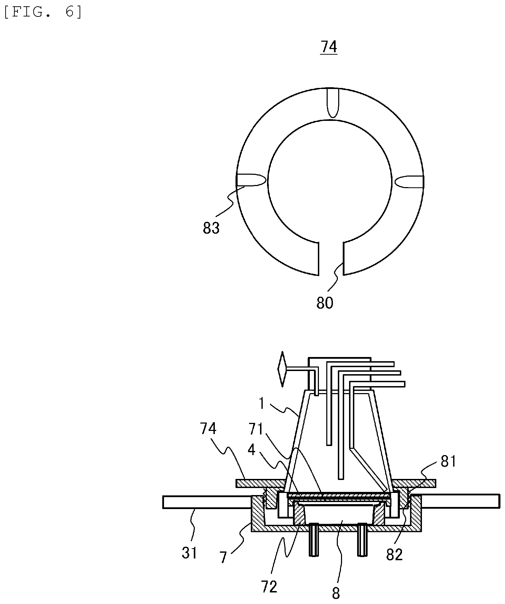

FIG. 6 shows another embodiment of the pressing jig 74. The features of the pressing jig 74 are as follows. The pressing jig 74 has a large opening in a central portion through which the culture vessel 1 penetrates. An opening portion 80 through which the tubes pass during placement is provided, and the pressing jig 74 is basically circular and has a male screw portion 81 facing the swing stage. On the other hand, a female screw portion 82 to be engaged with the male screw portion 81 is provided on an inner side surface of the ventilation adapter 7.

In the placement method, after the four tubes pass through the opening portion 80 above the culture vessel 1, the pressing jig 74 is placed in the ventilation adapter 7, and the male screw portion 81 is rotated by using a recessed portion shape 83 of the pressing jig 74 to couple to the screw portion. When a load is applied to the gas holding space forming portion 72 and the perforated bottom surface and the upper surface of the ventilation adapter 7 come into close contact with each other, the coupling of the screw portions is completed. The feature of the pressing jig 74 is that the pressing jig 74 can be produced by a set of component plates, and when the pressing jig 74 is closed, the pressing jig 74 can be in contact the vessel at equal intervals on the circumference of the vessel and press the vessel horizontally with a constant force. With this pressing force, the gas holding space forming portion 72 is pressed more firmly, and the ventilation adapter 7 can be placed with a stronger closing force.

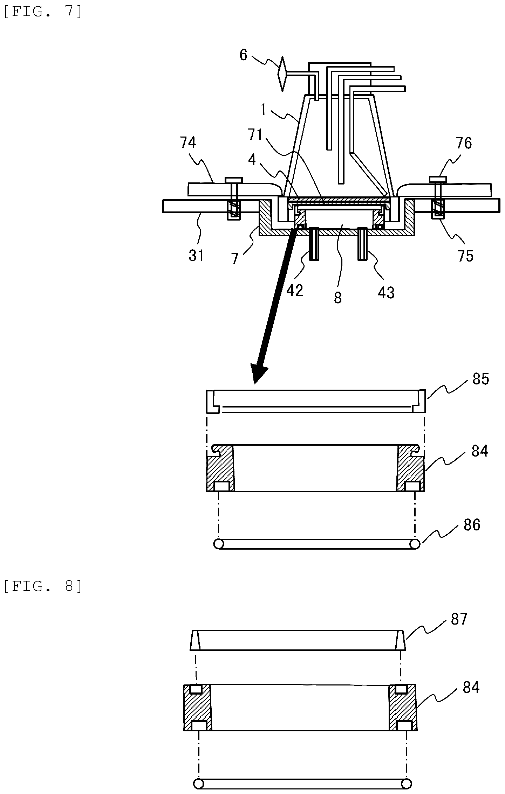

FIG. 7 shows another embodiment of the gas holding space forming portion 72. In the configuration of this embodiment, a groove is formed from a side portion to an upper portion of a packing holder 84 made of a material (hard material) having low flexibility such as metal or hard plastic, and an L-shaped packing 85 to be in contact with the perforated bottom surface 71 is fitted into the groove to be integrated (the L-shaped packing 85 is also referred to as a second airtight sealing portion). A downward groove is formed in a lower portion of the packing holder 84, and an O-ring 86 to be in contact with an inner upper surface of the ventilation adapter 7 is fitted into the groove to be integrated (the O-ring 86 is also referred to as a first airtight sealing portion). The packing holder 84 can be fixed inside the ventilation adapter 7 by a plurality of screws (not shown), and basically, the packing holder 84 is pressed against the ventilation adapter 7 by the screw force, and the O-ring 86 is elastically deformed to maintain the airtightness of the ventilation space 8. On the other hand, the L-shaped packing 85 comes into close contact with the perforated bottom surface 71 and is elastically deformed by the downward pressing by the pressing jig 74, so that the airtightness of the ventilation space 8 is maintained.

The feature of this method is as follows. An elastic member is used only in a portion where airtightness is necessary, and when the elastic member is consumed with the number of times of closing of the vessel and the airtightness is reduced, the airtightness is restored by replacing only the elastic member. Therefore, the manufacturing costs are lower and the running costs can also be lower than in the case where the whole is formed of rubber or the like having flexibility.

FIG. 8 shows another embodiment of the gas holding space forming portion 72. In the configuration of this embodiment, a groove is formed in an upper portion of the packing holder 84, and a trapezoidal packing 87 to be in contact with the perforated bottom surface 71 is fitted into the groove to be integrated. The difference between this method and the L-shaped packing 85 is that a direction of elastic deformation in close contact with the perforated bottom surface 71 due to downward pressing by the pressing jig 74 is downward in the case of the trapezoidal packing as compared with the case where the L-shaped packing is laterally deformed from above. The elastic members repel each other only in one direction, and therefore, stronger airtightness can be obtained, and the ventilation space 8 can be more strongly maintained in airtightness.

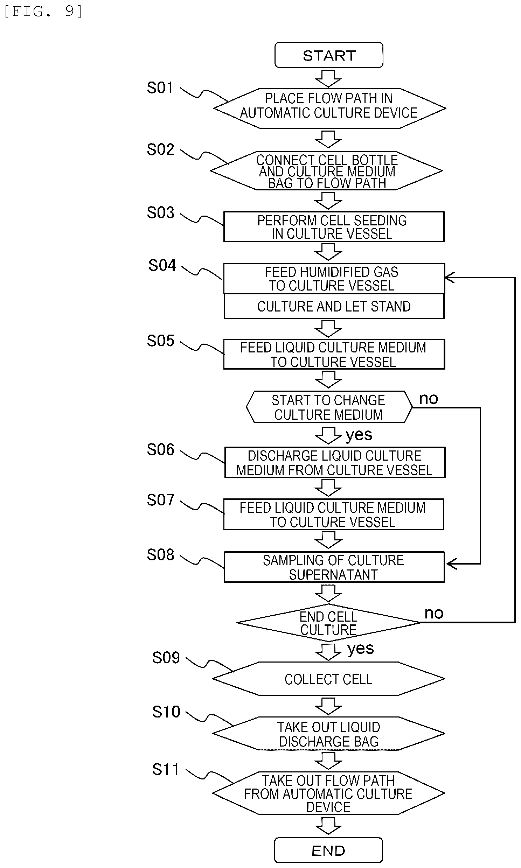

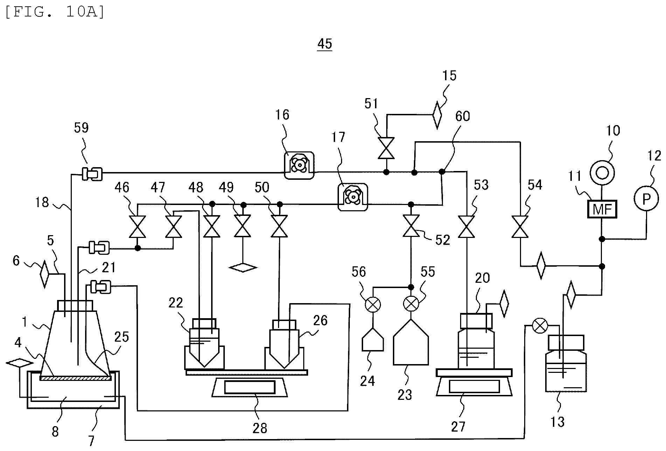

FIG. 9 is a flowchart of an overall operation of cell culture in a cell culture device 100 shown in FIG. 10A. Hereinafter, the flow of cell culture using the cell culture device 100 will be described with reference to FIGS. 10A to 10G.

FIG. 10A is a view showing an example of a flow path circuit 45 including a closed culture vessel according to Embodiment 1. The same elements as those in the embodiment shown in FIG. 1 are denoted by the same reference signs, and the specific solenoid valves 19-1 to 19-9 for the solenoid valve 19 are denoted by the reference signs 46 to 54.

The rubber tube connected to the supernatant collection bag 23 is provided with a manual valve 55 by which the tube can be manually opened and closed, and the rubber tube connected to the supernatant analysis bag 24 is provided with a manual valve 56. The reference sign 59 denotes a joint component, which is a component by which the joining and disconnection of pipelines are enabled by using male and female joints. The reference sign 60 denotes a branching portion, which is a branching point of tubes joined using a T-shaped connection component.

The flow path circuit 45 shown here can be integrally attached to and detached from the device body shown in Embodiment 1 except for the solenoid valve 19, the weight sensor (electronic balance) 28, the gas flow rate control unit 11, and the pressure sensor 12 in the pumps 16 and 17 as mechanical elements, and can be applied to cell culture by sterilizing only the flow path circuit.

In FIG. 9, after "START", the flow path is placed in the cell culture device 100 (S01). Then, the cell seeding bottle 22 for holding the separately prepared cell suspension, the culture medium bottle 20 for holding the culture medium, and the cell collection bottle 26 are connected to the flow path (S02).

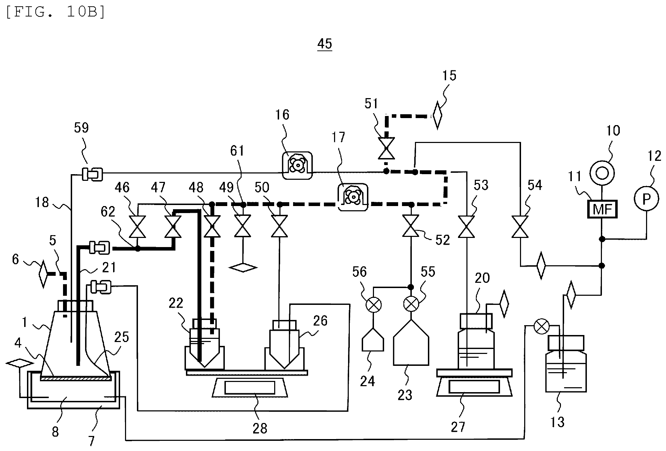

FIG. 10B shows a step of cell seeding according to Embodiment 1. In an initial state, since the pumps are stopped and the roller is stopped by clamping the rubber tube, the pumps 16 and 17 are closed as valves. The solenoid valve is closed by clamping the rubber tube. A predetermined amount of a cell suspension is held in the cell seeding bottle 22, and the cell seeding bottle 22 is placed in the weight sensor 28. The culture vessel 1 is horizontally placed on the ventilation adapter 7 in an empty state. At the start of cell seeding, the solenoid valve 47, the solenoid valve 48, the solenoid valve 54, and the solenoid valve 51 are opened, and a pipeline from a liquid discharge pipe 21 of the culture vessel 1 to the CO₂ gas vent filter 15 is opened.

Next, when the pump 17 is operated and a gas is fed from one of pipelines of the cell seeding bottle 22 to apply pressure to the cell suspension inside the bottle, the cell suspension passes through a pipeline and is fed from the liquid discharge pipe 21 to the culture vessel 1 (S03). A flow state of the liquid at this time is indicated by a solid line, and a flow state of the gas is indicated by a broken line (the same applies hereinafter).

Next, when a predetermined amount of the cell suspension has been moved, the solenoid valve 47 is closed, the solenoid valve 49 is opened, and the pump 17 is stopped simultaneously. At this time, the cell suspension is temporarily stopped in the pipeline by closing the solenoid valve 47, the gas pressurizing the cell seeding bottle 22 is discharged to the vent filter opened from a branching portion 61 to the outside, and the movement of the liquid is stopped.

Next, when the solenoid valves 48 and 49 are closed, the solenoid valve 46 is opened, and the pump 17 is operated, the cell suspension in the pipeline closer to the culture vessel than a branching portion 62 is fed to the culture vessel 1. Thereafter, when the pump 17 is stopped and the solenoid valves 47, 48, and 49 are opened, the cell suspension in the pipeline returns to the cell seeding bottle 22 due to the difference in height, there is no liquid in the pipeline, and all the solenoid valves are closed to end the cell seeding step (S03).

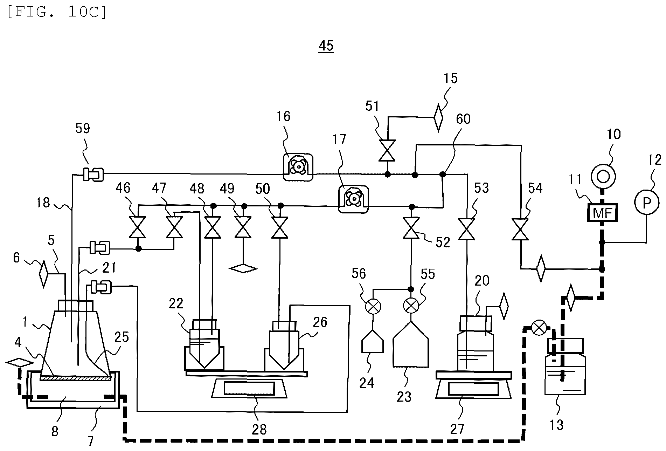

FIG. 10C shows a step of exchanging gas in the culture vessel 1 in Embodiment 1. In the initial state, the solenoid valve is closed by closing the rubber tube. Water is held in the humidification bottle 13, and an opening of one long pipe is provided at a bottom portion of the vessel. The gas flow rate control unit 11 and the gas cylinder 10 are connected to one end of the long pipe.

An opening of a short tube in the humidification bottle 13 is provided at an upper portion of the vessel, and the ventilation adapter is connected to one end of the short tube. When the gas flow rate control unit 11 is operated, the gas controlled at a predetermined air feeding amount is fed into the humidification bottle, humidified, and fed from the humidification bottle. Next, a gas concentration of the ventilation space 8 of the ventilation adapter 7 is increased and maintained at a predetermined gas concentration, and therefore, the gas exchange in the culture vessel 1 is continuously performed (S04).

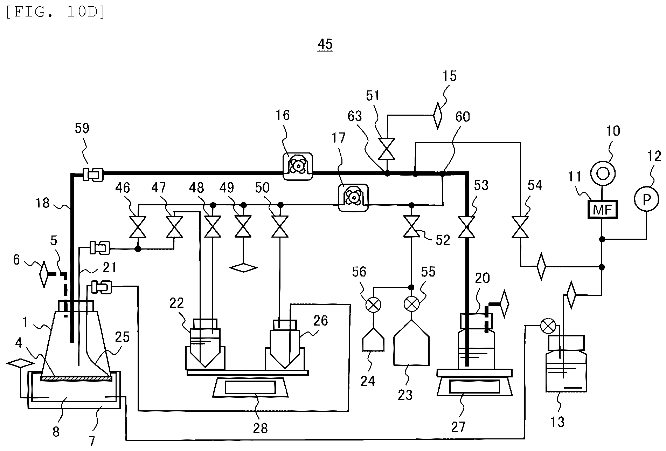

FIG. 10D shows the step of adding the culture medium to the culture vessel 1 in Embodiment 1. In an initial state, the pump is stopped, and the solenoid valve is closed by closing the rubber tube. The culture medium is held in the culture medium bottle 20, and the culture medium bottle 20 is placed on the weight sensor 27. First, the solenoid valve 53 is opened to open a pipeline from the liquid feeding pipe 18 of the culture vessel 1 to the culture medium bottle 20. Next, when the pump 16 is operated, the culture medium passes through the pipeline and is fed from the liquid feeding pipe 18 to the culture vessel 1.

Next, when a predetermined liquid amount of the culture medium has been moved, the solenoid valve 51 is opened, and the pump 16 is stopped simultaneously. At this time, the flow of the culture medium is temporarily stopped in the pipeline by the stop of the pump 16, and the culture medium in the pipeline close to the culture medium bottle 20 returns to the inside of the culture medium bottle 20 due to the difference in height after the outside air enters from the vent filter opened from a branching portion 63 to the outside.

Next, when the solenoid valve 53 is closed and the pump 16 is operated, the culture medium in the pipeline closer to the culture vessel than the branching portion 63 is fed to the culture vessel 1. Thereafter, when the pump 16 is stopped, there can be no liquid in the pipeline, and all the solenoid valves are closed to end the culture medium addition step (S05).

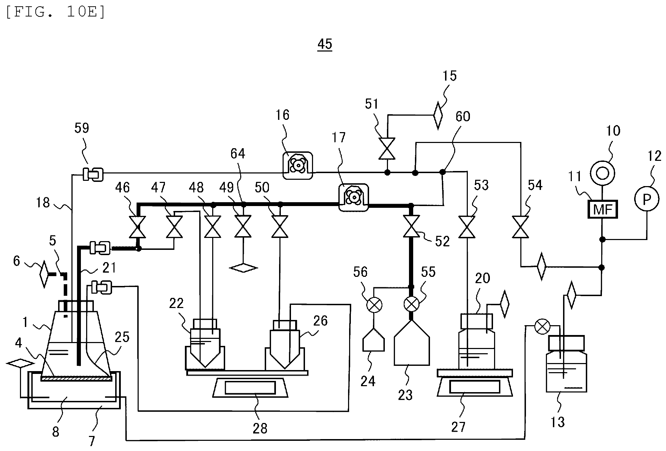

FIG. 10E shows a step of discharging the culture medium in culture medium change in the culture vessel 1 in Embodiment 1. In an initial state, the pump is stopped, and the solenoid valve is closed by closing the rubber tube. The culture medium is held in the culture vessel 1, and the supernatant collection bag 23 is empty. First, the solenoid valve 46, the solenoid valve 52, and the manual valve 55 are opened, and a pipeline from the liquid discharge pipe 21 of the culture vessel 1 to the supernatant collection bag 23 is opened. Next, when the pump 17 is operated, the culture medium is fed from the culture vessel 1 through the liquid discharge pipe 21 and reaches the supernatant collection bag 23.

Next, when the solenoid valve 49 is opened, the culture medium in the pipeline closer to the culture vessel 1 than the branching portion 64 (the same as the branching portion 61 in FIG. 10B) is fed to the culture vessel 1 by the difference in height. Thereafter, when the solenoid valve 46 is closed and the pump 17 is operated, the outside air is introduced from the vent filter, and the culture medium in the pipeline reaches the supernatant collection bag 23. All the solenoid valves are closed, and the culture medium discharge step ends (S06). Next, by performing the step of the culture medium addition to the culture vessel 1 described with reference to FIG. 10D in the same manner, the culture medium can be replaced (S07). After the culture medium is changed, the culture vessel 1 is swung by the swing mechanism 30 to mix a new culture medium with cells, thereby promoting the cell culture.

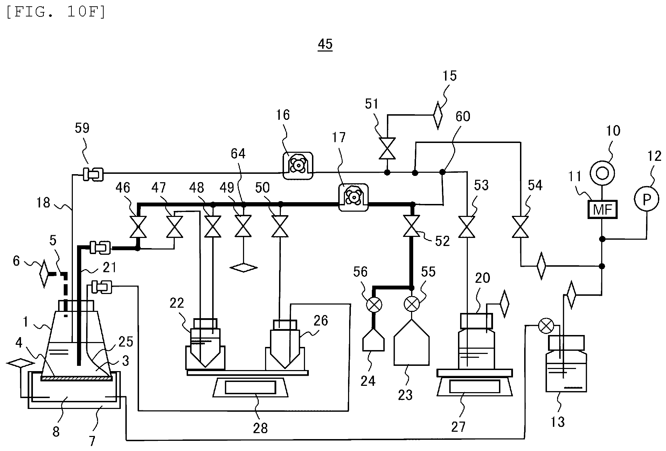

FIG. 10F shows a step of sampling the supernatant from the culture vessel 1 in Embodiment 1. In an initial state, the pump is stopped, and the solenoid valve is closed by closing the rubber tube. The culture medium is held in the culture vessel 1, and the supernatant analysis bag 24 is empty. First, the solenoid valve 46, the solenoid valve 52, and the manual valve 56 are opened, and a pipeline from the liquid discharge pipe 21 of the culture vessel 1 to the supernatant analysis bag 24 is opened. Next, when the pump 17 is operated, the culture medium is fed from the culture vessel 1 through the liquid discharge pipe 21 and reaches the supernatant collection bag 23.

Next, when the solenoid valve 49 is opened, the culture medium in the pipeline closer to the culture vessel 1 than the branching portion 64 is fed to the culture vessel 1 by the difference in height. Thereafter, when the solenoid valve 46 is closed and the pump 17 is operated, the outside air is introduced from the vent filter, and the culture medium in the pipeline reaches the supernatant analysis bag 24. All the solenoid valves are closed, and the supernatant sampling step ends (S08).

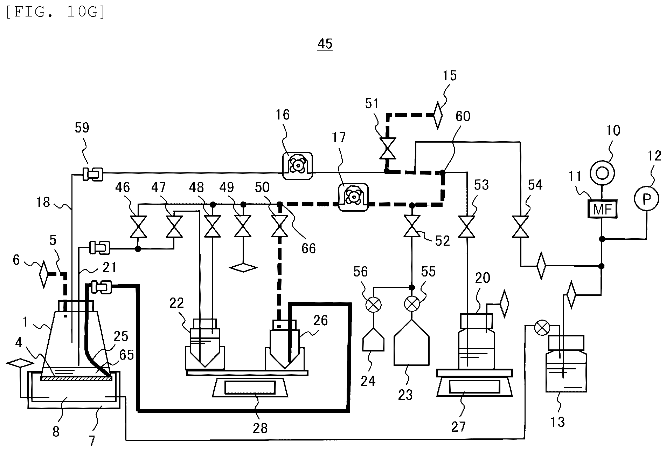

A step of collecting cells from the culture vessel 1 in Embodiment 1 will be described with reference to FIGS. 10E and 10G. After the cells have sufficiently grown, the cells settle to the bottom surface of the culture vessel and float thereon. Similarly to the supernatant collection step according to FIG. 10E, the supernatant is collected from the culture vessel 1 in the same flow path circuit, and the supernatant collection is continued until the culture medium 3 is below the height of an opening of the liquid discharge pipe 21. Accordingly, an abundance ratio of the cells to the culture medium 3 in the culture vessel 1 can be increased. Next, the culture vessel is swung by the above-described swing mechanism 30, and the culture medium and the cells are stirred to obtain a cell suspension.

Next, the step of collecting cells from the culture vessel 1 will be described with reference to FIGS. 10G and 10A. In an initial state, the pump is stopped, and the solenoid valve is closed by closing the rubber tube. A cell suspension 65 is held in the culture vessel 1, and the cell collection bottle 26 is empty.

Next, the solenoid valves 50 and 51 are opened, and a pipeline from the collection pipe 25 of the culture vessel 1 and the cell collection bottle 26 and a pipeline to the CO₂ gas vent filter 15 opened to the outside air are opened. Next, when the pump 17 is operated, the cell suspension 65 is fed from the culture vessel 1 through the collection pipe 25 and reaches the cell collection bottle 26.

Next, when the total amount of the cell suspension 65 has been moved to the cell collection bottle 26, the solenoid valve 49 is opened, and the pump 17 is stopped simultaneously. At this time, the outside air enters from the vent filter, opened to the outside, through a branching portion 66, the pressure inside the cell collection bottle 26 becomes a constant pressure, and the liquid feeding stops. All the solenoid valves are closed, and the cell collection step ends (S09).

After collecting the cells, a waste liquid bag is taken out from the flow path (S10), the flow path is taken out from the automatic culture device (S11), and all the steps of the automatic culture are completed.

As described above, the automatic culture device according to the present embodiment can perform culture while being reduced in the size thereof. It is possible to reduce a risk of occurrence of rust on mechanical elements such as a swing mechanism of a culture vessel used for a culture operation and a camera observation mechanism for cells, and to mount such mechanical elements. In addition, by the partial supply of the humidified gas to the culture vessel by the ventilation adapter, the risk of microorganism generation in the entire incubator can be reduced. In the present embodiment, the invention has been described using the humidified gas. However, when the influence of evaporation of the culture medium 3 through the ventilation surface 4 on the culture result is small, a dry gas instead of the humidified gas may be supplied to the ventilation surface 4. The supply of the dry gas can be implemented by directly connecting the flow rate control unit 11 and the ventilation adapter 7 without passing through the humidification bottle 13 shown in FIGS. 1 and 10. In this case, the influence on the mechanical element and the risk of microorganism generation can be further reduced, and there is an effect of saving labor in maintenance such as replenishment of water to the humidification bottle.

### Reference Signs List

1: culture vessel
2: cell
3: culture medium
4: ventilation surface
5: pressure regulating pipe
6: vent filter
7: ventilation adapter
8: ventilation space
9: gas supply unit
10: gas cylinder
11: flow rate control unit
12: pressure sensor
13: humidification bottle 13
14: CO₂ Sensor
15: CO₂ gas vent filter
16, 17: pump
18: liquid feeding pipe
19: solenoid valve
20: culture medium bottle
21: aspirating tube (liquid discharge pipe)
22: cell seeding bottle
23: supernatant collection bag
24: supernatant analysis bag
25: cell collection pipe
26: cell collection bottle
28: weight sensor
29: fluid control unit
30: swing mechanism
71: perforated bottom surface
72: gas holding space forming portion
73: gas flow meter
74: pressing jig

## Claims

1. An automatic culture device comprising:
a ventilation adapter configured to supply, in a state in which a culture vessel configured to culture cells is placed thereon, a gas containing CO₂ to the culture vessel;
a gas supply unit configured to supply the gas containing CO₂ to the ventilation adapter; and
a gas holding space forming portion configured to airtightly hold the gas containing CO₂ supplied from the gas supply unit when the culture vessel is placed at a predetermined position of the ventilation adapter, an upper portion of the gas holding space forming portion directly or indirectly supporting at least a part of a gas exchange membrane provided on a lower side of the culture vessel, and a lower end portion of the gas holding space forming portion coming into contact with at least a part of an upper surface of the ventilation adapter.

2. The automatic culture device according to claim 1, wherein
the gas holding space forming portion has a substantially cylindrical shape and has a structure in airtight contact with an inner peripheral surface of a skirt portion surrounding a periphery of the gas exchange membrane provided on the lower side of the culture vessel.

3. The automatic culture device according to claim 1, further comprising:
a pressing jig configured to press the culture vessel against the gas holding space forming portion.

4. The automatic culture device according to claim 1, wherein
at least one of a portion of the gas holding space forming portion directly or indirectly supporting at least a part of the gas exchange membrane and a portion of the gas holding space forming portion coming into contact with at least a part of the upper surface of the ventilation adapter is made of a member having flexibility.

5. The automatic culture device according to claim 1, wherein
the gas holding space forming portion is made of a hard material having a substantially cylindrical shape, and
at least one of a portion directly or indirectly supporting at least a part of the gas exchange membrane and a portion coming into contact with at least a part of the upper surface of the ventilation adapter is made of a member having flexibility.

6. The automatic culture device according to claim 1, wherein
the gas holding space forming portion is made of a hard material having a substantially cylindrical shape, and includes, in at least a part of a portion of the gas holding space forming portion to be in contact with the upper surface of the ventilation adapter, a first airtight sealing portion interposed between the gas holding space forming portion and the upper surface of the ventilation adapter, the first airtight sealing portion being configured to improve airtightness between the gas holding space forming portion and the ventilation adapter.

7. The automatic culture device according to claim 1, wherein
the gas holding space forming portion includes, in a portion directly or indirectly supporting at least a part of the gas exchange membrane, a second airtight sealing portion interposed between the gas holding space forming portion and the portion directly or indirectly supporting at least a part of the gas exchange membrane, the second airtight sealing portion being configured to improve airtightness between the gas holding space forming portion and the portion directly or indirectly supporting at least a part of the gas exchange membrane.

8. The automatic culture device according to claim 6, wherein
the first airtight sealing portion is an O-ring to be fitted into a groove provided in a part of a lower surface of the gas holding space forming portion.

9. The automatic culture device according to claim 7, wherein
the second airtight sealing portion is made of a ring-shaped member having an engagement portion to be engaged with a part of an upper end portion of the gas holding space forming portion.

10. The automatic culture device according to claim 9, wherein
the ring-shaped member has a substantially L-shaped cross section, and has a structure in which a short side portion of the L-shape engages with a groove portion provided in the gas holding space forming portion.

11. The automatic culture device according to claim 9, wherein
the ring-shaped member has a substantially I-shaped cross section, and has a structure in which a lower end portion of the I-shape engages with a groove portion provided in the gas holding space forming portion.

12. The automatic culture device according to claim 3, wherein
the pressing jig includes a structure that is openable and closable around a rotation shaft extending in a vertical direction.

13. The automatic culture device according to claim 3, wherein
the pressing jig has a hole configured to fix the ventilation adapter and the pressing jig via a fastener inserted into a hole provided in a support member supporting the ventilation adapter.

14. The automatic culture device according to any one of claims 1 to 13, wherein,
when the culture vessel is placed at a predetermined position of the ventilation adapter, culture is performed under a pressure higher than an atmospheric pressure of a place where the culture vessel is placed, the pressure being a pressure of a space formed by the gas holding space forming portion and airtightly holding the gas containing CO₂.

15. A cell culture method using an automatic culture device including a ventilation adapter configured to supply, in a state in which a culture vessel configured to culture cells is placed thereon, a gas containing CO₂ to the culture vessel, a gas supply unit configured to supply the gas containing CO₂ to the ventilation adapter, and a gas holding space forming portion configured to airtightly hold the gas containing CO₂ supplied from the gas supply unit when the culture vessel is placed at a predetermined position of the
